# EUROPÄISCHE PATENTSCHRIFT

(11) **EP 0 504 818 B1**
(45) Veröffentlichungstag und Bekanntmachung des Hinweises auf die Patenterteilung: **16.07.1997**
(21) Anmeldenummer: 92104613.2
(22) Anmeldetag: 17.03.1992
(51) Int. Cl.: C12P 17/12, C12N 1/20

(54) **Mikrobiologisches Verfahren zur Herstellung von 6-Hydroxypicolinsäure**
Microbiological process for the preparation of 6-hydroxy picolinic acid
Procédé microbiologique pour la préparation de l'acide 6-hydroxy picolinique

(30) Priorität: 18.03.1991 CH 812/91
(43) Veröffentlichungstag der Anmeldung: 23.09.1992
(73) Patentinhaber: LONZA AG, CH-3945 Gampel/Wallis (CH)
(72) Erfinder: Kiener, Andreas, Dr., Visp (Kanton Wallis) (CH)
(74) Vertreter: Weinhold, Peter, Dr.

(56) Entgegenhaltungen:
- EP-A- 0 152 948
- EP-A- 0 187 680
- JOURNAL OF BIOTECHNOLOGY Bd. 8, Nr. 1, Mai 1988, AMSTERDAM NL Seiten 87 - 95 JACQUES MAUGER ET AL. 'Nitrile hydratase-catalyzed production of isonicotinamide,picolinamide and pyrazinamide from 4-cynopyridine,2- cyanopyridine and cyanopyrazine in Rhodococcus rhodochrous J1.'
- CHEMICAL ABSTRACTS, vol.88, no.1, 2. Januar 1978, Columbus, Ohio, US; abstract no. 2900j, SHUKLA, O. ET AL. 'Microbial transformation of pyridine derivatives: alpha-picolinate metabolism by a gram-negative coccus.' Seite 270

## Beschreibung

Die Erfindung betrifft ein neues mikrobiologisches Verfahren zur Herstellung von 6-Hydroxypicolinsäure ausgehend von 2-Cyanpyridin sowie neue für das Verfahren geeignete Mikroorganismen.

6-Hydroxypicolinsäure wird beispielsweise zur Herstellung von 2-Oxypyrimidin verwendet (Berichte der Deutschen Chemischen Gesellschaft, 1912, 45, S. 2456-2467), welches wiederum ein wichtiges Zwischenprodukt zur Herstellung von Pharmazeutika ist.

Bekannt ist, dass Mikroorganismen der Gattung Bacillus Picolinsäure zur 6-Hydroxypicolinsäure hydroxylieren (O.Shukla und S.M. Kaul, Indian J. of Biochemistry and Biophysics, Vol.10, S176-178, 1973; O.Shukla et al., Indian J. of Biochemistry and Biophysics, Vol.14, S.292-295, 1977).

Ein grosser Nachteil dieses Verfahrens ist, dass die weitere Metabolisierung der 6-Hydroxypicolinsäure nur mit dem Inhibitor Natriumarsenit unterbunden werden kann, wobei damit auch das Wachstum der Mikroorganismen gehemmt wird. Ein weiterer Nachteil besteht darin, dass nicht ausschliesslich 6-Hydroxypicolinsäure gebildet wird, sondern ein Gemisch von 3,6-Dihydroxypicolinsäure und 6-Hydroxypicolinsäure.

R.L. Tate und J.C. Ensign, Can. J. Microbiol., Vol.20, S.695-702, 1974, beschreiben die Hydroxylierung von Picolinsäure mit Mikroorganismen der Gattung Arthrobacter.
Nachteile dieses Verfahrens sind, dass diese Mikroorganismen Picolinsäure nicht ausschliesslich als Kohlenstoff-, Stickstoff- und Energiequelle nutzen können, sondern bei der Hydroxylierung Hefe-Extrakt anwesend sein muss, der zu unerwünschten Verunreinigungen des Produktes führen kann.
Ein weiterer Nachteil liegt darin, dass die 6-Hydroxypicolinsäure nur bei geringem Sauerstoffgehalt gebildet wird, wobei sich die Mikroorganismen nicht in der Wachstumsphase befinden und dadurch wenig Produkt gebildet wird.

Aufgabe der vorliegenden Erfindung war es, diese Nachteile zu beseitigen und ein einfaches, wirtschaftliches mikrobiologisches Verfahren zur Herstellung von 6-Hydroxypicolinsäure, ausgehend von 2-Cyanpyridin, zur Verfügung zu stellen.

Diese Aufgabe wurde mit einem neuen Verfahren gemäss Patentanspruch 3, das mit neuen Mikroorganismen gemäss Patentanspruch 1 durchgeführt wird, gelöst.

Erfindungsgemäss sind alle Mikroorganismen geeignet, die befähigt sind mit 2-Cyanpyridin als einziger Kohlenstoff-, Stickstoff- und Energiequelle zu wachsen und dieses als Substrat in 6-Hydroxypicolinsäure zu überführen.

Diese Mikroorganismen sind Bestandteil der Erfindung und können mit Hilfe üblicher mikrobiologischer Techniken, beispielsweise aus Kläranlagen,mit 2-Cyanpyridin als Wachstumssubstrat selektioniert und isoliert werden.

Der Begriff "Mikroorganismen, die befähigt sind, mit 2-Cyanpyridin als einziger Kohlenstoff-,Stickstoff- und Energiequelle zu wachsen", umfasst sowohl Mischungen von Mikroorganismen als auch Rein-Isolate dieser Mikroorganismen, die unter sterilen oder unsterilen Fermentationsbedingungen eingesetzt werden können.

Zweckmässig werden die Mikroorganismen Alcaligenes faecalis und deren Deszendenten und Mutanten eingesetzt. Diese wurden bei der Deutschen Sammlung für Mikroorganismen und Zellkulturen GmbH (DSM), Mascheroderweg 1b, D-3300 Braunschweig am 31.1.1991 mit der Bezeichnung DSM 6335 hinterlegt.

Wissenschaftliche Beschreibung von Alcaligenes faecalis (DSM 6335)

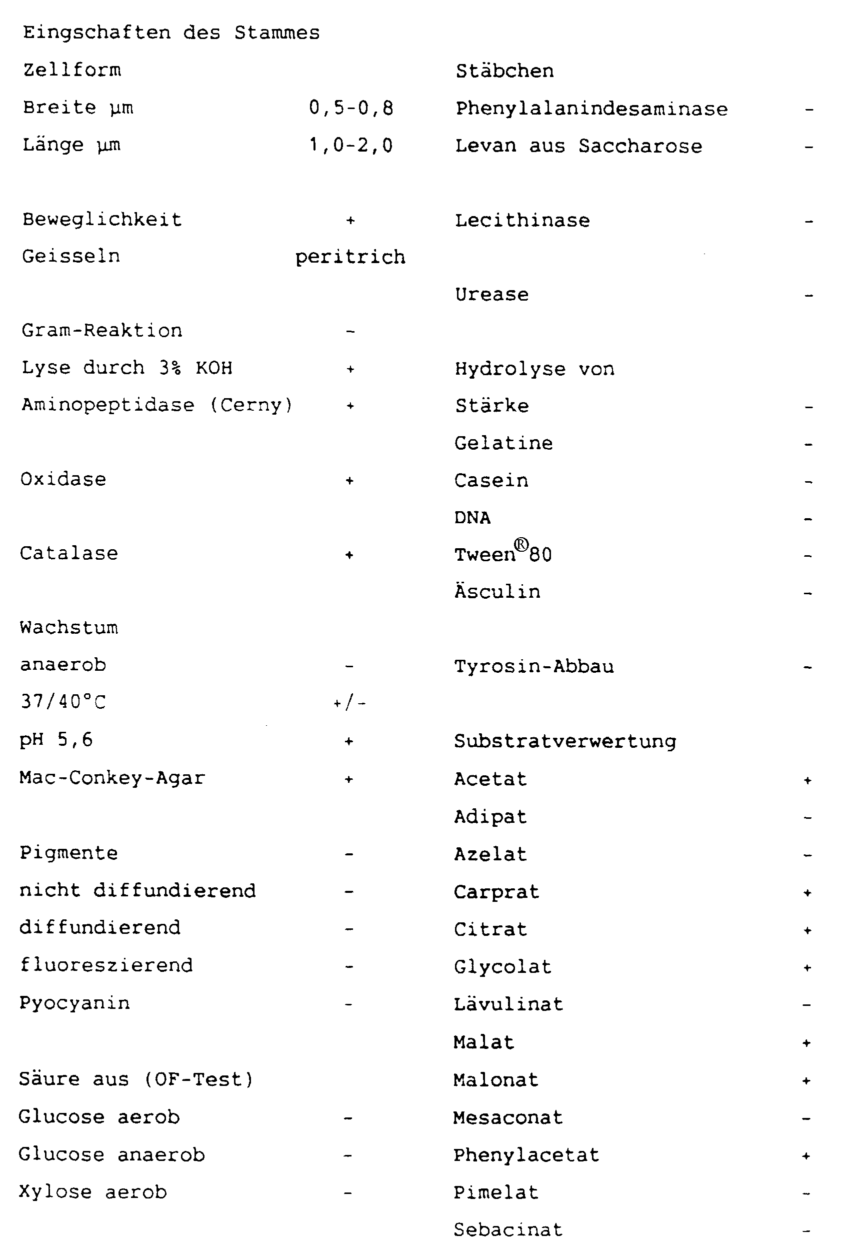

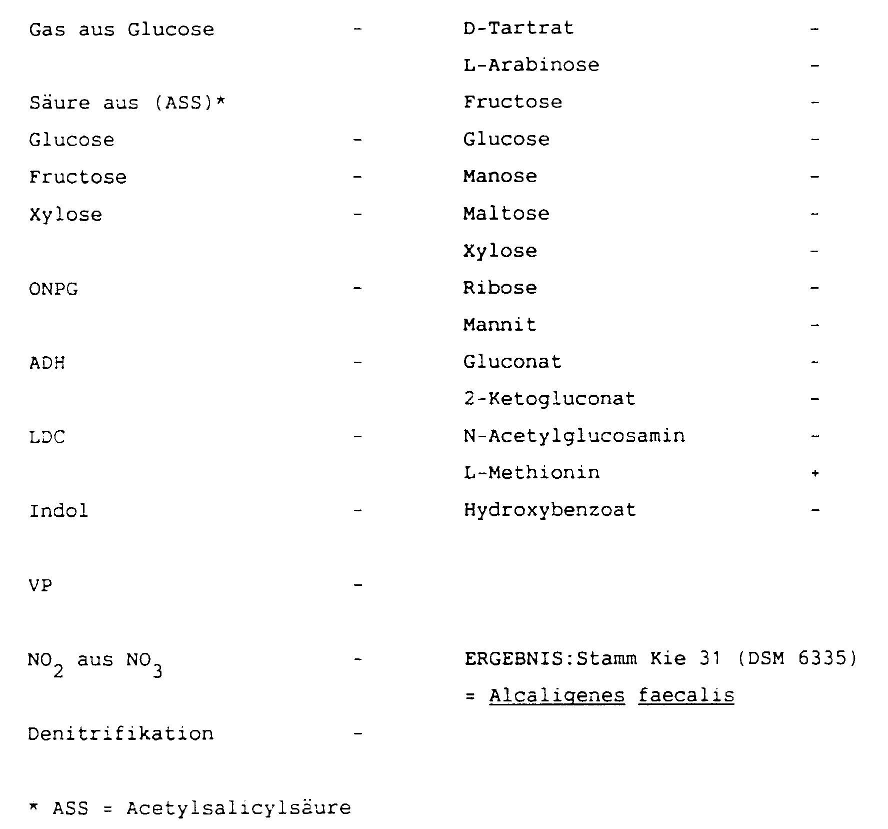

Das Verfahren zur Herstellung von 6-Hydroxypicolinsäure wird erfindungsgemäss derart durchgeführt, dass man 2-Cyanpyridin mit einem dieser Mikroorganismen zu 6-Hydroxypicolinsäure biotransformiert, wobei letzteres im Medium akkumuliert wird.

Vor der eigentlichen Umsetzung werden diese Mikroorganismen üblicherweise kultiviert (angezogen) und die wirksamen Enzyme der Mikroorganismen zweckmässig mit 2-Cyanpyridin induziert.

Üblicherweise erfolgt die Kultivierung (Anzucht) und die Induktion mit 2-Cyanpyridin in einer Konzentration von 0,01 bis 20 Gew.%, vorzugsweise in einer Konzentration von 0,1 bis 1 Gew.%.

Anschliessend können die Mikroorganismen entweder vor der Substratzugabe (2-Cyanpyridin) mittels üblichen Trennverfahren geerntet werden, oder das Substrat (2-Cyanpyridin) kann direkt zu den Mikroorganismen zugegeben werden.

Für das eigentliche Verfahren wird dann zweckmässig die Zellsuspension auf eine optische Dichte bei 650 nm von 1 bis 100, vorzugsweise auf eine optische Dichte von 5 bis 80, eingestellt.

Als Medium können die in der Fachwelt üblichen angewendet werden, vorzugsweise wird eines der Medien verwendet, deren Zusammensetzung in Tabelle 1 und 2 angegeben ist.

Das Substrat (2-Cyanpyridin) kann zur Produktion von 6-Hydroxypicolinsäure einmalig oder kontinuierlich zugegeben werden. Zweckmässig erfolgt die Substratzugabe so, dass die Substratkonzentration im Medium 20 Gew.%, vorzugsweise so, dass die Substratkonzentration 10 Gew.% nicht übersteigt.
Üblicherweise erfolgt die Umsetzung von 2-Cyanpyridin zur 6-Hydroxypicolinsäure mit ruhenden Zellen.

Der pH-Wert der Umsetzung liegt zweckmässig in einem Bereich von 4 bis 10, vorzugsweise in einem Bereich von 5 bis 9.

Zweckmässig wird die Umsetzung bei einer Temperatur von 10 bis 50°C, vorzugsweise bei einer Temperatur von 20 bis 40°C,durchgeführt.

Nach einer üblichen Umsetzungsdauer von 1 bis 100 Stunden kann 6-Hydroxypicolinsäure beispielsweise durch Ansäuern der zellfreien Fermentationslösung isoliert werden.

### Beispiel 1

### Isolierung von 2-Cyanpyridin-metabolisierenden Mikroorganismen

Aerobe 2-Cyanpyridin-metabolisierende Mikroorganismen wurden im A+N-Medium (Tabelle 1) mit Zusatz von 0,1% (w/v) 2-Cyanpyridin als einziger Kohlenstoff- und Energiequelle angereichert. Die allgemeinen Techniken zur Isolation von Mikroorganismen sind beispielsweise in "G.Drews, Mikrobiologisches Praktikum, 4.Aufl., Springer Verlag, 1983" beschrieben.
Als Inokolum wurden Proben aus Kläranlagen verwendet.
Die Anreicherungen wurden in Schüttelkolben bei 30°C angezogen. Nach dreimaligem Überimpfen in frisches Medium wurden die Anreicherungen auf dem gleichen Medium mit Zusatz von 16 g Agar pro Liter ausgestrichen und bei 30°C inkubiert. Nach mehrmaligem Ausstreichen auf Agarmedium konnten Reinkulturen isoliert werden.

**Tabelle 1**

| A+N-Medium | |
|---|---|
| Zusammensetzung: | Konzentration (mg/l) |
| (NH₄)₂SO₄ | 2000 |
| Na₂HPO₄ | 2000 |
| KH₂PO₄ | 1000 |
| NaCl | 3000 |
| MgCl₂·6H₂O | 400 |
| CaCl₂·2H₂O | 14.5 |
| FeCl₃·6H₂O | 0.8 |
| Pyridoxal-Hydrochlorid | 10·10⁻³ |
| Riboflavin | 5·10⁻³ |
| Nicotinsäureamid | 5·10⁻³ |
| Thiamin-Hydrochlorid | 2·10⁻³ |
| Biotin | 2·10⁻³ |
| Panthothensäure | 5·10⁻³ |
| p-Aminobenzoat | 5·10⁻³ |
| Folsäure | 2·10⁻³ |
| Vitamin B12 | 5·10⁻³ |
| ZnSO₄·7H₂O | 100·10⁻³ |
| MnCl₂·4H₂O | 90·10⁻³ |
| H₃BO₃ | 300·10⁻³ |
| CoCl₂·6H₂O | 200·10⁻³ |
| CuCl₂·2H₂O | 10·10⁻³ |
| NiCl₂·H₂O | 20·10⁻³ |
| Na₂MoO₄·H₂O | 30·10⁻³ |
| EDTANa₂·H₂O | 5·10⁻³ |
| FeSO₄·H₂O | 2·10⁻³ |
| (pH der Lösung wurde auf 7.0 eingestellt) | |

### Beispiel 2

### Umsetzung von 2-Cyanpyridin zu 6-Hydroxypicolinsäure

a) Alcaligenes faecalis DSM-Nr. 6335 wurde im A+N-Medium (Tabelle 1) unter Zusatz von 0,1% (w/v) 2-Cyanpyridin in einem Fermenter bei pH 7 und bei einer Temperatur von 30°C angezogen.
Anschliessend wurden die Zellen abzentrifugiert , im A+N-Medium resuspendiert und auf eine optische Dichte bei 650 nm von 10 eingestellt.
Diese Zellsuspension wurde in einen Schüttelkolben gegeben und mit 0,1 mol/l (10,4 g/l) 2-Cyanpyridin versetzt. Nach einer Inkubation von 16 h bei 30°C auf einer Schüttelmaschine konnten mit analytischen Methoden 0,04 mol/l (5,5 g/l) 6-Hydroxypicolinsäure in der zellfreien Lösung nachgewiesen werden, was eine Ausbeute von 40%, bezogen auf eingesetztes 2-Cyanpyridin, entsprach.
b) Alcaligenes faecalis DSM 6335 wurde in einem Mineralsalzmedium (Tabelle 2) unter Zusatz von 0,1% (w/v) 2-Cyanpyridin in einem Fermenter (Arbeitsvolumen 5,5 l) bei pH 7 und bei einer Temperatur von 30°C angezogen. Zur pH-Regelung wurden 3 mol/l Natriumhydroxid und 8,5% (v/v) Phosphorsäure verwendet.
Während des Wachstums wurde zusätzlich 2-Cyanpyridin zum Fermenter gegeben, bis nach 24 h Wachstum die optische Dichte bei 650 nm 5,1 betrug.
Insgesamtwurden während der Wachstumsphase 35 g 2-Cyanpyridin metabolisiert.
Zur Herstellung von 6-Hydroxypicolinsäure wurde die Mikroorganismensuspension mit 2-Cyanpyridin (220 g) versetzt. Nach einer weiteren Inkubation von 18 h wurden aus der zellfreien Lösung 108 g 6-Hydroxypicolinsäure isoliert, was einer Ausbeute von 37%, bezogen auf eingesetztes 2-Cyanpyridin, entsprach.

**Tabelle 2:**

| Zusammensetzung des Minersalzmediums | |
|---|---|
| - MgCl₂·6H₂O | 0,8 g/l |
| - CaCl₂ | 0,16 g/l |
| - Na₂SO₄ | 0,25 g/l |
| - KH₂PO₄ | 0,4 g/l |
| - Na₂HPO₄ | 0,9 g/l |
| - SLF | 1 ml/l |
| - FeEDTA | 15 ml/l |
| Zusammensetzung der Spurenelemente (SLF) im Mineralsalzmedium | |
| - KOH | 15 g/l |
| - EDTANa₂·2H₂O | 100 g/l |
| - ZnSO₄ ·7H₂O | 9 g/l |
| - MnCl₂·4H₂O | 4 g/l |
| - H₃BO₃ | 2,7 g/l |
| - CoCl₂·6H₂O | 1,8 g/l |
| - CuCl₂·2H₂O | 1,5 g/l |
| - NiCl₂·6H₂O | 0,18 g/l |
| - Na₂MoO₄·2H₂O | 0,2 g/l |
| Zusammensetzung von FeEDTA | |
| - EDTA Na₂·2H₂O | 5 g/l |
| - FeSO₄·7H₂O | 2 g/l |
| (Der pH der Lösung wurde auf 7,0 eingestellt) | |

## Patentansprüche

1. Mikroorganismen, dadurch gekennzeichnet, daß sie befähigt sind, mit 2-Cyanpyridin als einziger Kohlenstoff-, Stickstoff- und Energiequelle zu wachsen und dieses als Substrat in 6-Hydroxypicolinsäure zu überführen.

2. Mikroorganismen nach Anspruch 1 mit der Bezeichnung Alcaligenes faecalis, hinterlegt bei der DSM mit der Nummer 6335, und deren Deszendenten und Mutanten, die befähigt sind, mit 2-Cyanpyridin als einziger Kohlenstoff-, Stickstoff- und Energiequelle zu wachsen und dieses als Substrat in 6-Hydroxypicolinsäure zu überführen.

3. Mikrobiologisches Verfahren zur Herstellung von 6-Hydroxypicolinsäure, dadurch gekennzeichnet, daß man 2-Cyanpyridin mit Mikroorganismen gemäß einem der Ansprüche 1 und 2 zu 6-Hydroxypicolinsäure biotransformiert, wobei letztere im Medium akkumuliert wird.

4. Verfahren nach Anspruch 3, dadurch gekennzeichnet, daß die wirksamen Enzyme der Mikroorganismen mit 2-Cyanpyridin induziert werden.

5. Verfahren nach mindestens einem der Ansprüche 3 und 4, dadurch gekennzeichnet, daß die Umsetzung unter einmaliger oder kontinuierlicher Substratzugabe erfolgt, so daß die Substratkonzentration 20 Gew.-% nicht übersteigt.

6. Verfahren nach mindestens einem der Ansprüche 3 bis 5, dadurch gekennzeichnet, daß man die Umsetzung bei einem pH von 4 bis 10 und einer Temperatur von 10 bis 50°C durchführt.

## Claims

1. Microorganisms, characterised in that they are capable of growth using 2-cyanopyridine as the sole source of oxygen, nitrogen and energy and of converting this as a substrate into 6-hydroxypicolinic acid.

2. Microorganisms according to claim 1 having the name Alcaligenes faecalis, deposited at the DSM (Deutsche Sammlung für Mikroorganismen und Zellkulturen GmbH) under the number 6335, and the descendants and mutants thereof, which are capable of growth using 2-cyanopyridine as the sole source of oxygen, nitrogen and energy and of converting this as a substrate into 6-hydroxypicolinic acid.

3. Microbiological process for the preparation of 6-hydroxypicolinic acid, characterised in that 2-cyanopyridine is biotransformed, using microorganisms according to one of claims 1 and 2, to 6-hydroxypicolinic acid, the latter being accumulated in the medium.

4. Process according to claim 3, characterised in that the active enzymes of the microorganisms are induced with 2-cyanopyridine.

5. Process according to at least one of claims 3 and 4, characterised in that the conversion is carried out with the single or continuous addition of the substrate in a manner such that the substrate concentration does not exceed 20 wt.%.

6. Process according to at least one of claims 3 to 5, characterised in that the conversion is carried out at a pH of from 4 to 10 and at a temperature of from 10°C to 50°C.

## Revendications

1. Microorganismes, caractérisés en ce qu'ils sont capables de croître avec la 2-cyanopyridine en tant que seule et unique source de carbone, d'azote et d'énergie et de transformer celle-ci en tant que substrat en l'acide 6-hydroxypicolinique.

2. Microorganismes selon la revendication 1, portant la désignation Alcaligenes faecalis, déposés auprès de l'Institut DSM sous le numéro 6335, et dont les descendants et mutants sont capables de croître avec la 2-cyanopyridine en tant que seule et unique source de carbone, d'azote et d'énergie et de transformer celle-ci en tant que substrat en l'acide 6-hydroxypicolinique.

3. Procédé microbiologique pour la préparation de l'acide 6-hydroxypicolinique, caractérisé en ce que l'on procède à la biotransformation de la 2-cyanopyridine avec des microorganismes selon l'une des revendications 1 et 2, en l'acide 6-hydroxypicolinique, ce dernier étant accumulé dans le milieu.

4. Procédé selon la revendication 3, caractérisé en ce que les enzymes efficaces des microorganismes sont induites avec la 2-cyanopyridine.

5. Procédé selon au moins l'une des revendications 3 et 4, caractérisé en ce que la réaction s'effectue avec l'addition du substrat en une seule fois ou de façon continue de façon que la concentration en substrat ne dépasse pas 20 % en poids.

6. Procédé selon au moins l'une des revendications 3 à 3, caractérisé en ce que l'on effectue la réaction à un pH de 4 à 10 et à une température de 10 à 50°C.
